# FASCICULE DE BREVET EUROPEEN

(11) **EP 1 233 743 B1**
(45) Date de publication et mention de la délivrance du brevet: **15.02.2006**
(21) Numéro de dépôt: 00969640.2
(22) Date de dépôt: 18.10.2000
(51) Int. Cl.: A61K 8/49, A61Q 5/10

(54) **COMPOSITIONS DE TEINTURE DES FIBRES KERATINIQUES CONTENANT DES 3-AMINO PYRAZOLO-[1,5-a]-PYRIDINES, PROCEDE DE TEINTURE, NOUVELLES 3-AMINO PYRAZOLO-[1,5-a]-PYRIDINES**
3-AMINOPYRAZOLO(1,5-A)PYRIDINE ENTHALTENDE ZUSAMMENSETZUNGEN ZUR FÄRBUNG VON KERATINISCHEN FÄSERN , FÄRBEVERFAHREN UND NEUE 3-AMINOPYRAZOLO(1,5-A)PYRIDINE
COMPOSITIONS FOR DYEING KERATINOUS FIBRES CONTAINING 3-AMINO PYRAZOLO-[1,5-A]-PYRIDINES, DYEING METHOD, NOVEL 3-AMINO PYRAZOLO-[1,5-A]-PYRIDINES

(30) Priorité: 19.11.1999 FR 9914582
(43) Date de publication de la demande: 28.08.2002
(73) Titulaire: L'OREAL, 75008 Paris (FR)
(72) Inventeur: BIRAULT, Véronique, SAFFRON WALDEN,ESSEX GRANDE-BRETAGNE (GB); LEDUC, Madeleine, Résidence Les Chèvrefeuilles, 75011 Paris (FR); TERRANOVA, Eric, F-92270 Bois Colombes (FR)
(74) Mandataire: Fevrier, Murielle Françoise E.
(86) Numéro de dépôt international: PCT/FR2000/002903
(87) Numéro de publication internationale: WO 2001/035917

(56) Documents cités:
- EP-A- 0 030 680
- EP-A- 0 299 209
- EP-A- 0 433 855
- EP-A- 0 904 769
- FR-A- 2 771 631
- US-A- 3 536 436
- US-A- 5 234 818
- US-A- 5 980 585

## Description

L'invention a pour objet de nouvelles compositions pour la teinture d'oxydation des fibres kératiniques comprenant au moins une 3-amino pyrazolo-[1,5-a]-pyridine à titre de base d'oxydation, le procédé de teinture mettant en oeuvre cette composition, de nouvelles 3-amino pyrazolo-[1,5-a]-pyridines, ainsi que leur utilisation pour la teinture d'oxydation des fibres kératiniques.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants. Ces composés ont pour point commun de posséder un groupement amino et un groupement hydroxyle ou deux groupements amino, ce qui leur confère leur caractère de base d'oxydation.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.
La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les brevets GB 1 026 978 et GB 1 153 196, d'utiliser des pyridines telles que la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, comme bases d'oxydation pour la teinture d'oxydation des fibres kératiniques.

La demanderesse vient maintenant de découvrir, de façon totalement inattendue et surprenante, une nouvelle famille de 3-amino pyrazolo-[1,5-a]-pyridine de formule (I) définie ci-après, pour partie nouveaux en soi, pouvant convenir pour une utilisation comme base d'oxydation, mais en outre permettant d'obtenir des compositions tinctoriales qui conduisent à des colorations puissantes même à pH neutre, et qui présentent une bonne tenue face aux agents extérieurs (lumière, intempéries, lavage, ondulation permanente, transpiration, frottements).

Ces découvertes sont à la base de la présente invention.

L'invention a donc pour premier objet l'utilisation pour la teinture d'oxydation des fibres kératiniques et en particulier des fibres kératiniques humaines telles que les cheveux, d'une composition comprenant, dans un milieu approprié pour la teinture, au moins une 3-amino pyrazolo-[1,5-a]-pyridine de formule (I) suivante à titre de base d'oxydation et/ou un de ses sels d'addition avec un acide ou avec une base : 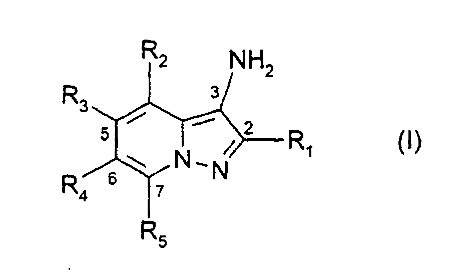
dans laquelle :
- R₁, R₂, R₃, R₄ et R₅, identiques ou différents, représentent un atome d'hydrogène ou d'halogène ; un radical -NHSO₃H ; un radical hydroxyle ; un radical (C₁-C₄)alkyle ; un radical (C₁-C₄)alkoxy ; un radical (C₁-C₄)alkylthio ; mono(C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical nitro ; un radical phényle ; un radical carbonyle ; un radical alkoxy(C₁-C₄)carbonyle ; un radical carboxamido ; un radical cyano ; un radical amino ; un radical sulfonyle; un radical -CO₂H, un radical -SO₃H ; un radical -PO₃H₂ ; un radical -PO₄H₂; ou un groupement de formule (II) suivante : 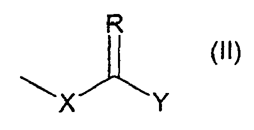 dans lequel R représente un atome d'oxygène ou d'azote, X représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino.

Dans les composés de formule (I) ci-dessus, l'expression alkyle utilisée pour les radicaux alkyle ainsi que pour les groupements comportant une partie alkyle, signifie une chaîne carbonée, linéaire ou ramifiée, comportant de 1 à 4 atomes de carbone, substituée ou non substituée par un ou plusieurs hétérocycles, ou par un ou plusieurs groupements phényle ou par un ou plusieurs groupes choisis parmi les atomes d'halogène tel le chlore, le brome, l'iode et le fluor ; les radicaux hydroxyle, alcoxyle, amino, carbonyle, carboxamido, sulfonyle, -CO₂H, -SO₃H, -PO₃H₂, -PO₄H₂, -NHSO₃H, sulfonamide, monoalkyl(C₁-C₄)amino, trialkyl(C₁-C₄)ammonium, ou bien encore par un radical dialkyl(C₁-C₄)amino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre.

De même, selon l'invention, l'expression alcoxy utilisée pour les radicaux alcoxy ainsi que pour les groupements comportant une partie alcoxy, signifie une chaîne O-carbonée, linéaire ou ramifiée, comportant de 1 à 4 de carbone, substituée ou non substituée par un ou plusieurs groupes choisis parmi les hétérocycles ; les atomes d'halogène tel le chlore, le brome, l'iode et le fluor ; les radicaux hydroxyle, amino, carbonyle, carboxamido, sulfonyle, -CO₂H, -SO₃H, -PO₃H₂, -PO₄H₂, -NHSO₃H, sulfonamide, monoalkyl(C₁-C₄)amino, trialkyl(C₁-C₄)ammonium, ou bien encore par un radical dialkyl(C₁-C₄)amino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre.

Selon l'invention, on entend par hétérocycle, un cycle aromatique ou non contenant 5, 6 ou 7 sommets, et de 1 à 3 hétéroatomes choisis parmi les atomes d'azote, de soufre et d'oxygène. Ces hétérocycles peuvent être condensés sur d'autres hétérocycles ou sur un groupement phényle. Ils peuvent être substitués par un atome d'halogène ; un radical (C₁-C₄)alkyle ; un radical (C₁-C₄)alkoxy ; un radical hydroxyle ; un radical amino ; un radical (C₁-C₄)alkylamino ; di(C₁-C₄) alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre. Ces hétérocycles peuvent, en outre, être quaternisés par un radical (C₁-C₄)alkyle.

Parmi ces hétérocycles, on peut notamment citer à titre d'exemple les cycles : thiadiazole, triazole, isoxazole, oxazole, azaphosphole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine, 3-(2-hydroxyéthyl)benzothiazol-3-ium, et 1-(2-hydroxyéthyl)-pyridinium.

Selon l'invention, on entend par phényle, un radical phényle non substitué ou substitué par un ou plusieurs radicaux cyano, carbonyle, carboxamido, sulfonyle, -CO₂H, -SO₃H, -PO₃H₂, -PO₄H₂, hydroxyle, amino, monoalkyl(C₁₋C₄)amino, ou dialkyl(C₁-C₄)amino dans lequel les deux groupements alkyle peuvent former, conjointement avec l'atome d'azote dudit groupement dialkyl(C₁-C₄)amino auquel ils sont liés, un cycle pouvant être interrompu par un ou plusieurs atomes d'azote, d'oxygène ou de soufre.

Parmi les groupements de formule (II) ci-dessus, on peut notamment citer les groupement acétamide, diméthylurée, O-méthylcarbamate, méthylcarbonate, N-diméthylcarbamate et les esters.

Parmi les composés de formule (I) ci-dessus, on préfère les 3-amino pyrazolo-[1,5-a]-pyridines répondant à la sous-formule (Ia) suivante, et leurs sels d'addition avec un acide ou avec une base : 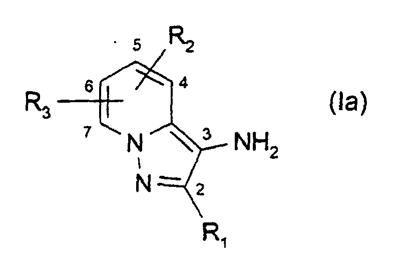
dans laquelle :
R₁, R₂, R₃, identiques ou différents, représentent un atome d'hydrogène ou d'halogène ; un radical hydroxyle ; un radical (C₁-C₄)alkyle ; un radical (C₁-C₄)alkylthio ; un radical (C₁-C₄)alkoxy ; un radical -NHSO₃H ; un radical amino ; un radical (C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle tel que défini précédemment ; un radical sulfonamide, un radical carbonyle, un radical alcoxy(C₁-C₄)carbonyle, un radical carboxamido, ou un groupement de formule (II) suivante : 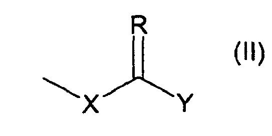 dans lequel R représente un atome d'oxygène ou d'azote, X représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino.

Parmi les 3-amino pyrazolo-[1,5-a]-pyridines de formule (I), utilisables à titre de base d'oxydation dans les compositions tinctoriales conformes à l'invention, on peut notamment citer :
- la pyrazolo[1,5-a]pyridin-3-ylamine ;
- la 2-acétylamino pyrazolo-[1,5-a] pyridin-3-ylamine ;
- la 2-morpholin-4-yl-pyrazolo[1,5-a]pyridin-3-ylamine ;
- l'acide 3-amino-pyrazolo[1,5-a]pyridin-2-carboxylique ;
- la 2-méthoxy-pyrazolo[1,5-a]pyridine-3-ylamino ;
- le (3-amino-pyrazolo[1,5-a]pyridine-7-yl)-méthanol ;
- le 2-(3-amino-pyrazolo[1,5-a]pyridine-5-yl)-éthanol ;
- le 2-(3-amino-pyrazolo[1,5-a]pyridine-7-yl)-éthanol ;
- le (3-amino-pyrazolo[1,5-a]pyridine-2-yl)-méthanol ;
- la 3,6-diamino-pyrazolo[1,5-a]pyridine ;
- la 3,4-diamino-pyrazolo[1,5-a]pyridine ;
- la pyrazolo[1,5-a]pyridine-3,7-diamine ;
- la 7-morpholin-4-yl-pyrazolo[1,5-a]pyridin-3-ylamine ;
- la pyrazolo[1,5-a]pyridine-3,5-diamine ;
- la 5-morpholin-4-yl-pyrazolo[1,5-a]pyridin-3-ylamine ;
- le 2-[(3-amino-pyrazolo[1,5-a]pyridin-5-yl)-(2-hydroxyéthyl)-amino]-éthanol ;
- le 2-[(3-amino-pyrazolo[1,5-a]pyridin-7-yl)-(2-hydroxyéthyl)-amino]-éthanol ;
- la 3-amino-pyrazolo[1,5-a]pyridine-5-ol ;
- la 3-amino-pyrazolo[1,5-a]pyridine-4-ol ;
- la 3-amino-pyrazolo[1,5-a]pyridine-6-ol ;
- la 3-amino-pyrazolo[1,5-a]pyridine-7-ol ;

et leurs d'addition avec un acide ou avec une base.

Pour leur grande majorité, les 3-amino pyrazolo-[1,5-a]-pyridines de formule (I) sont des composés connus dans le domaine pharmaceutique, et sont décrites notamment dans le brevet US 5,457,200. Ces composés peuvent être préparés selon des méthodes de synthèse bien connues dans la littérature et telles que décrites par exemple dans le brevet US 5,457,200.

La ou les 3-amino pyrazolo-[1,5-a]-pyridines de formule (I) ci-dessus et/ou le ou leurs sels d'addition avec un acide ou une base représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 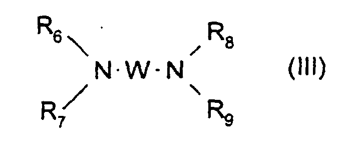
dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

Selon une forme de réalisation préférée, la composition de teinture d'oxydation conforme à l'invention renferme en outre un ou plusieurs coupleurs de façon à modifier ou à enrichir en reflets les nuances obtenues en mettant en oeuvre les composés de formule (I).

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtal, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition.

Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut encore contenir, en plus des colorants définis ci-dessus, au moins une base d'oxydation additionnelle, et qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques différentes des 3-amino pyrazolo-[1,5-a]-pyridines de formule (I) utilisées conformément à l'invention.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.
Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques différents des composés de formule (I) conformes à l'invention, les dérivés pyrimidiniques et les dérivés pyrazoliques.

Parmi les dérivés pyridiniques différents des composés de formule (I) conformes à l'invention, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, la ou les bases d'oxydation additionnelles représentent de préférence de 0,0005 à 12 % en poids environ du poids total de la composition tinctoriale, et encore plus préférentiellement de 0,005 à 6 % en poids environ de ce poids.

D'une manière générale, les sels d'addition avec un acide utilisables dans le cadre des compositions tinctoriales de l'invention (composés de formule (1), (la), bases d'oxydation additionnelles et coupleurs) sont notamment choisis parmi les chlorhydrates, les bromhydrates, les sulfates, les citrates, les succinates, les tartrates, les lactates, les phosphates et les acétates. Les sels d'addition avec une base utilisables dans le cadre des compositions tinctoriales de l'invention sont notamment ceux obtenus avec la soude, la potasse, l'ammoniaque ou les amines.

La composition tinctoriale conforme à l'invention peut en outre renfermer un ou plusieurs colorants directs pouvant notamment être choisis parmi les colorants nitrés de la série benzénique.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.
Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes parmi lesquelles on peut citer les peroxydases, les oxydo-réductases à 2 électrons telles que les uricases et les oxygénases à 4 électrons comme les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Certains composés de formule (I), utilisés à titre de base d'oxydation dans le cadre de la présente invention, sont nouveaux et, à ce titre, constituent un autre objet de l'invention.

Ces nouvelles 3-amino pyrazolo-[1,5-a]-pyridines, et leurs sels d'addition avec un acide ou avec une base, répondent à la formule (I') suivante : 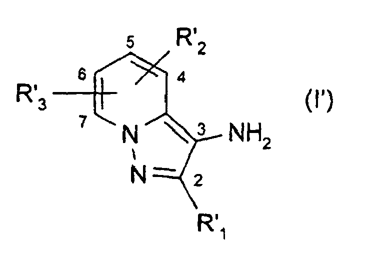
dans laquelle :
- R'₁ représente un atome d'hydrogène ou d'halogène ; un radical hydroxyle ; (C₁-C₄)alkyle ; un radical (C₁-C₄)alkylthio ; un radical (C₁-C₄)alkoxy ; un radical amino ; un radical (C₁-C₄)alkylamino ; un radical di(C₁-C₄)alkylamino dans lequel les deux groupements alkyle, conjointement avec l'atome d'azote auquel ils sont liés, peuvent former un cycle pouvant être interrompu par un ou plusieurs atomes d'azote, d'oxygène ou de soufre ; un hétérocycle ; ou un groupement de formule (II') suivante : 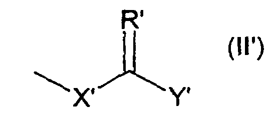 dans lequel R' représente un atome d'oxygène ou d'azote, X' représente un atome d'oxygène, un groupement NH ou NH(C₁-C₄)alkyle, et Y' représente un radical hydroxyle, amino, alkyle en C₁-C₄, (C₁-C₄)alkoxy, (C₁-C₄)alkylamino, ou di(C₁-C₄)alkylamino.
- R'₂ et R'₃, identiques ou différents, représentent un atome d'hydrogène ; un atome d'halogène ; un radical nitro ; un hétérocycle ; un radical NHSO₃H ; un radical sulfonamide ; un radical (C₁-C₄)alkyle substitué par un ou plusieurs radicaux identiques ou différents et choisis parmi les hétérocycles, les radicaux -CO₂H, -SO₃H, -PO₃H₂, -PO₄H₂, hydroxyle, tri(C₁-C₄)alkylammonium, -NHSO₃H, sulfonamide, amino, (C₁-C₄)alkylamino et di(C₁-C₄)alkylamino dans lequel les deux radicaux alkyle peuvent former conjointement avec l'atome d'azote auquel ils sont liés un cycle pouvant être interrompu par un ou plusieurs atomes d'azote, de soufre ou d'oxygène ; un radical (C₁-C₄)alkylthio substitué par un ou plusieurs radicaux hydroxyle, amino substitué ou non, ou par un ou plusieurs groupements -CO₂H, -SO₃H, -PO₃H₂ ou PO₄H₂, ou par un ou plusieurs hétérocycles ; un radical (C₁-C₄)alkoxy substitué par un ou plusieurs radicaux hydroxyle, amino substitué ou non, ou par un ou plusieurs groupements -CO₂H, -SO₃H, -PO₃H₂ ou PO₄H₂, ou par un ou plusieurs hétérocycles ; un radical amino substitué par un ou deux radicaux (C₁-C₄)alkyle, ledit ou lesdits radicaux alkyle étant eux-mêmes substitués par un radical amino substitué ou non, tri(C₁-C₄)alkylammonium, -CO₂H, -SO₃H, -PO₃H₂, -PO₄H₂, -NHSO₃H, ou par un hétérocycle ;

étant entendu que :
- au moins un des radicaux R'₁ à R'₃ est différent d'un atome d'hydrogène ;
- les radicaux R'₂ et R'₃ ne peuvent représenter simultanément un atome d'hydrogène ;
- lorsque R'₁ représente un hétérocycle, alors R'₂ et R'₃ sont différents d'un atome d'halogène et d'un atome d'hydrogène ;
- lorsque R'₁ représente un atome d'hydrogène, et que l'un des radicaux R'₂ ou R'₃ représente également un atome d'hydrogène, alors l'autre radical R'₂ ou R'₃ est différent d'un radical hydroxyméthyle en position 7 ou d'un radical β-hydroxyéthyle en position 7 ou 5 ;
- lorsque R'₁ représente un radical méthoxy et que l'un des radicaux R'₂ ou R'₃ représente un atome d'hydrogène, alors l'autre radical R'₂ ou R'₃ est différent d'un atome de chlore.

Dans les composés de formule (I') ci-dessus, l'expression hétérocycle signifie un cycle aromatique ou non contenant 5, 6 ou 7 sommets, et de 1 à 3 hétéroatomes choisis parmi les atomes d'azote, de soufre et d'oxygène. Ces hétérocycles peuvent être condensés sur d'autres hétérocycles ou sur un groupement phényle. Ils peuvent être substitués par un atome d'halogène ; un radical (C₁-C₄)alkyle ; un radical (C₁-C₄)alkoxy ; un radical hydroxyle ; un radical amino ; un radical (C₁-C₄)alkylamino ; di(C₁-C₄) alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre. Ces hétérocycles peuvent, en outre, être quaternisés par un radical (C₁-C₄)alkyle.

Parmi ces hétérocycles, on peut notamment citer à titre d'exemple les cycles : thiadiazole, triazole, isoxazole, oxazole, azaphosphole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine, 3-(2-hydroxyéthyl)benzothiazol-3-ium, et 1-(2-hydroxyéthyl)-pyridinium.

Parmi les groupements de formule (II') ci-dessus, on peut notamment citer les groupements acétamide, diméthylurée, O-méthylcarbamate, méthylcarbonate, N-diméthylcarbamate et les esters.

Parmi les 3-amino pyrazolo-[1,5-a]-pyridines de formule (I') ci-dessus, on peut notamment citer :
- la 5-pyridin-4-yl-pyrazolo[1,5-a]pyridin-3-ylamine ;
- le 4-(3-amino-pyrazolo[1,5-a]pyridin-5-yl)-1-méthyl-pyridinium ;
- le 4-(3-amino-pyrazolo[1,5-a]pyridin-5-yl)-1-(2-hydroxyéthyl)-pyridinium ;
- le (3-amino-pyrazolo[1,5-a]pyridin-2-yl)-pyridin-2-yl-méthanol ;
- le 2-[(3-amino-pyrazolo[1,5-a]pyridin-2-yl)-hydroxyméthyl]-1-méthyl-pyridinium ;
- le 2-[(3-amino-pyrazolo[1,5-a]pyridin-2-yl)-hydroxyméthyl]-1-(2-hydroxyéthyl)-pyridinium ;
- la N7-(2-imidazo-1-yl-propyl)-pyrazolo[1,5-a]pyridin-3,7-diamine ;
- le 3-[2-(3-amino-pyrazolo[1,5-a]pyridin-7-ylamino)-propyl]-1-méthyl-3H-imidazol-1-ium ;
- le 3-[2-(3-amino-pyrazolo[1,5-a]pyridin-7-ylamino)-propyl]-1-(2-hydroxyéthyl)-3H-imidazol-1-ium ;
- la N5-(3-imidazo-1-yl-propyl)-pyrazolo[1,5-a]pyridin-3,5-diamine ;
- le 3-[3-(3-amino-pyrazolo[1,5-a]pyridin-5-ylamino)-propyl]-1-méthyl-3H-imidazol-1-ium ;
- le 3-[3-(3-amino- pyrazolo[1,5-a]pyridin-5-ylamino)-propyl]-1-(2-hydroxyéthyl)-3H-imidazol-1-ium ;

et leurs sels d'addition avec un acide ou avec une base.
Les sels d'addition avec un acide des composés de formule (I') ci-dessus, sont de préférence choisis parmi les chlorhydrates, les bromhydrates, les sulfates, les citrates, les succinates, les tartrates, les lactates, les phosphates et les acétates. Les sels d'addition avec une base des composés de formule (I') ci-dessus sont de préférence choisis parmi ceux obtenus avec la soude, la potasse, l'ammoniaque ou les amines.

Ces nouvelles 3-amino pyrazolo-[1,5-a]-pyrimidines de formule (I') ci-dessus, ainsi que plus généralement les 3-amino pyrazolo-[1,5-a]-pyrimidines de formule (I) décrite précédemment, peuvent être préparées selon des méthodes connues et décrites dans la littérature, et par exemple selon le schéma de synthèse suivant : 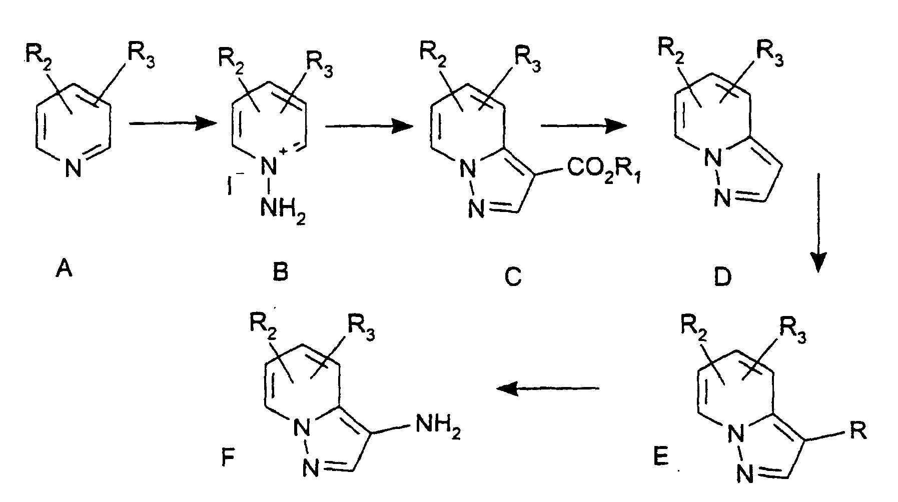 selon lequel un composé **A** est aminé par exemple avec du -NH₂SO₃H ou du NH₂SO₃Ms (o-mésytilène sulfonyl hydroxylamine) pour donner un composé **B**, avec transformation du sel de sulfate en sel de iodure. Ces réactions d'amination sont décrites notamment dans J. Org. Chem., 33, (1968), 3766 ; Chem. Pharm. Bull., 22, (1974), 482 ; Tet. Lett., (1972,) 4133 ; Synthesis, (1977), 1 ; ou bien encore dans Bull. Chem. Soc. Jpn, 49, (1976), 1980.

Le composé **C** peut ensuite être obtenu par cyclisation 1-3 dipolaire du composé **B** avec du propiolate de méthyle ou d'éthyle. Cette réaction de cyclisation est décrite dans Liebigs Ann. Chem., (1977), 498 ; Tet. Lett., (1962), 387 ; Arch. Pharm., 321, (1988), 505 ; J. Het. Chem., 18, (1981), 1149 ; Het., 24, (1986), 3411 ; Biorg. Med. Chem. Lett., 3, (1993),1477.

Le composé **C** est transformé en composé **D**, après hydrolyse de l'ester pour donner l'acide correspondant, suivi d'une décarboxylation, voir Liebigs Ann. Chem., 1977, 498; J. Het. Chem., 18, (1981), 1149.

L'introduction d'un radical R désignant un groupe nitro, nitroso ou arylazo à partir du composé **D** pour donner le composé **E** se fait selon des méthodes décrites dans la littérature. La nitration peut par exemple être réalisée avec de l'acide nitrique, de l'acide nitrique mélangé avec de l'acide sulfurique ou de l'acide nitrique mélangé avec de l'acide acétique. La nitrosation peut par exemple être réalisée avec de l'acide nitreux, L'introduction d'un radical arylazo peut se faire par réaction du sel d'aryldiazonium sur le composé **D**.

Ces méthodes sont décrites dans «Nitration Method and Mechanism» G Olah, R. Malhotra, S. Narang, VCH Publishers ; Houben-Weyl, Methoden der Organishe Chemie, Vol 10/1 et 10/3 ; US 5 457 200 ; J. Heterocycl. Chem., (1974), 11, 223-225.

Les groupements nitro, nitroso et arylazo sont ensuite réduits pour conduire à un composé **F** selon des méthodes décrites dans la littérature. La réduction peut par exemple s'effectuer avec du zinc dans l'acide acétique, l'acide acétique glacial et dithionite de sodium, du chlorure d'étain dans un acide, ou bien encore par hydrogénation Catalytique. Voir notamment Houben-Weyl. Methoden der Organishe Chemie, Vol 10/1 et 10/3 ; US 5,457,200.

Les pyridines de départ (composés A) ont été décrites ou peuvent être préparées par analogie avec des composés connus. Concernant la préparation de pyridines voir Comprehensive Heterocyclic Chemistry Il vol. 5, A. Katritzky, C. Rees, E. Scriven.

On pourra préparer les composés de la formule générale (I) ou (I') substitués en position 2, par analogie avec J. Het. Chem., 1975, 481; Chem. Pharm. Bull, 21, 1973, 2146.

L'invention a enfin pour objet une composition de teinture des fibres kératiniques comprenant, dans un milieu approprié, des 3-amino pyrazolo-[1,5]-pyridines de formule (I'), ainsi que de leurs sels d'addition avec un acide ou avec une base, à titre de base d'oxydation pour la teinture d'oxydation des fibres kératiniques et en particulier des fibres kératiniques humaines telles que les cheveux.

Les exemples qui suivent sont destinés à illustrer l'invention.

### EXEMPLES DE SYNTHESE

### EXEMPLE 1 : Synthèse du dichlorhydrate de 3,4-diamino-pyrazolo-[1,5-a]-pyridine

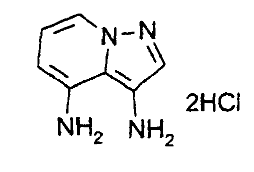

A une suspension de 11,1 g de chlorure d'étain dans 80 ml d'acide chlorhydrique concentré, ont été ajoutés 0,7 g de 3,4-dinitro-pyrazolo-[1,5-a]-pyridine. La réaction a été suivie par chromatographie sur couche mince (CCM). Le pH du milieu réactionnel a été ajusté à 12 par de la soude. La phase aqueuse a été extraite à l'acétate d'éthyle, et la phase organique a été séchée sur sulfate de sodium. La phase organique a été acidifiée avec 3 ml d'éthanol chlorhydrique HCl 2.5N. Le précipité a été filtré. On a obtenu 0,55 g de dichlorhydrate de pyrazolo[1,5-a]pyridine-3,4-diamine dont l'analyse RMN ¹H (DMSO-d₆, 400MHz) (δ ppm) était la suivante :
RMN H1 (DMSO d₆) : 6.82 (d, 1H) ; 6.97 (d,1H) ; 8.18 (s, 1H) ; 8.36 (d,1H).

### EXEMPLE 2 : Synthèse de la 3,6-diamino-pyrazolo-[1,5-a]-pyridine

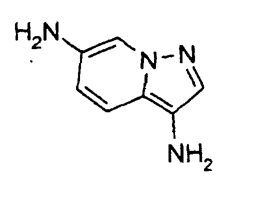

### a) Première étape : Préparation de la 3,6-dinitro-pyrazolo-[1,5-a]-pyridine par nitration de la pyrazolo-[1,5-a]-pyridine selon J. Heterocycl. Chem., (1974), 11, 223-225.

A une solution de 4,5 g de pyrazolo-[1,5-a]-pyridine dans 20 ml d'acide sulfurique concentré, a été ajouté 3,75 ml d'acide nitrique concentré. La réaction a été suivie par chromatographie phase vapeur. Après 4 heures de réaction, on a ajouté 1 ml d'acide nitrique. Le mélange réactionnel a été jeté sur 200 ml de glace, et le précipité a été filtré.

Le produit a été obtenu en mélange avec la 3,4-dinitro-pyrazolo-[1,5-a]-pyridine et isolé par flash chromatographie sur silice. L'analyse RMN ¹H (DMSO-d₆, 400MHz) (δ ppm) était la suivante :
RMN H1 (DMSO d6) : 10.15 (d, 1H); 9.16 (s,1H); 8.50 (dd, 1H); 8.36 (d,1H).

### b) Deuxième étape : Préparation de la pyrazolo-[1,5-a]-pyridine-3,6-diamine

La réduction de la 3,6-dinitro-pyrazolo-[1,5-a]-pyridine a été effectuée par du zinc, dans un mélange éthanol/eau.
SM (ionisation chimique à pression atmosphérique) : MH⁺ 149.1.

### EXEMPLES 1 à 5 DE TEINTURE EN MILIEU ALCALIN

On a préparé les compositions tinctoriales, conformes à l'invention, suivantes :

| **COMPOSITION** | **1** | **2** | **3** | **4** | **5** |
|---|---|---|---|---|---|
| Dichlorhydrate de 3,4-diaminopyrazolo-[1,5-a]pyridine (Base d'oxydation de formule (I)) | 3.10⁻³ mole | - | - | - | - |
| Chlorhydrate de pyrazolo-[1,5-a]pyridin-3-ylamine (Base d'oxydation de formule (I)) | - | 3.10⁻³ mole | 3.10⁻³ mole | 3.10⁻³ mole | - |
| Chlorhydrate de 2-acétylamino pyrazolo-[1,5-a] pyridin-3-ylamine (Base d'oxydation de formule (I)) | - | - | - | - | 3.10⁻³ mole |
| 2,4-diamino 1-(β-hydroxyéthyloxy) benzène (coupleur) | 3.10⁻³ mole | - | 3.10⁻³ mole | - | 3.10⁻³ mole |
| 3-amino phénol (coupleur) | - | 3.10⁻³ mole | - | - | - |
| 6-hydroxy indole (coupleur) | - | - | - | 3.10⁻³ mole | - |
| Support de teinture commun n°1 | (*) | (*) | (*) | (*) | (*) |
| Eau déminéralisée q.s.p. | 100 g | 100 g | 100 g | 100 g | 100 g |

| | | | | | |
|---|---|---|---|---|---|
| (*) Support de teinture commun n°1 : - Alcool éthyfique à 96° 18 g - Métabisulfite de sodium en solution aqueuse à 35% 0,68 g - Sel pentasodique de l'acide diéthylènetriaminopentacétique 1,1 g - Ammoniaque à 20% 10,0 g - Eau déminéralisée qs 100 g | | | | | |

Au moment de l'emploi, on a mélangé poids pour poids chacune des compositions tinctoriales ci-dessus avec une solution de peroxyde d'hydrogène à 20 volumes (6 % en poids) de pH 3.

Chacun des mélanges obtenu a été appliqué sur des mèches de cheveux gris naturels à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **PH de teinture** | **Nuance obtenue** |
|---|---|---|
| **1** | 10 ± 0,2 | Blond cendré |
| **2** | 10 ± 0,2 | Acajou irisé |
| **3** | 10 ± 0,2 | Violine |
| **4** | 10 ± 0,2 | Doré cuivré |
| **5** | 10 ± 0,2 | Cendré légèrement violacé |

### EXEMPLES 6 à 10 DE TEINTURE EN MILIEU NEUTRE

On a préparé les compositions tinctoriales, conformes à l'invention, suivantes :

| **COMPOSITION** | **6** | **7** | **8** | **9** | **10** |
|---|---|---|---|---|---|
| Dichlorhydrate de 3,4-diaminopyrazolo-[1,5-a]pyridine (Base d'oxydation de formule (I)) | 3.10⁻³ mole | - | - | - | - |
| Chlorhydrate de pyrazolo-[1,5-a]pyridin-3-ylamine (Base d'oxydation de formule (I)) | - | 3.10⁻³ mole | 3.10⁻³ mole | 3.10⁻³ mole | - |
| Chlorhydrate de 2-acétylamino pyrazolo-[1,5-a] pyridin-3-ylamine (Base d'oxydation de formule (I)) | - | - | - | - | 3.10⁻³ mole |
| 2,4-diamino 1-(β-hydroxyéthyloxy) benzène (coupleur) | 3.10⁻³ mole | - | 3.10⁻³ mole | - | 3.10⁻³ mole |
| 5-N-(β-hydroxyéthyl)amino 2-méthyl phénol (coupleur) | - | 3.10⁻³ mole | - | - | - |
| 6-hydroxy indole (coupleur) | - | - | - | 3.10⁻³ mole | - |
| Support de teinture commun n°2 | (**) | (**) | (**) | (**) | (**) |
| Eau déminéralisée q.s.p. | 100 g | 100 g | 100 g | 100 g | 100 g |

| | | | | | |
|---|---|---|---|---|---|
| (**) Support de teinture commun n°2 : - Ethanol à 96° 18 g - Tampon K₂HPO₄ /KH₂PO₄ (1,5 M / 1 M) 10 g - Métabisulfite de sodium 0,68 g - Sel pentasodique de l'acide diéthylènetriaminopentacétique 1,1 g | | | | | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris permanentés à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **6** | 5,7 ± 0,2 | Cendré légèrement mat |
| **7** | 5,7 ± 0,2 | Doré cuivré |
| **8** | 5,7 ± 0,2 | Irisé violine |
| **9** | 5,7 ± 0,2 | Doré acajou |
| **10** | 5,7 ± 0,2 | Cendré très légèrement irisé |

## Revendications

1. Utilisation pour la teinture d'oxydation des fibres kératiniques, d'une composition comprenant, dans un milieu approprié pour la teinture, au moins une 3-amino pyrazolo-[1,5-a]-pyridine de formule (I) suivante à titre de base d'oxydation et/ou un de ses sels d'addition avec un acide ou avec une base : 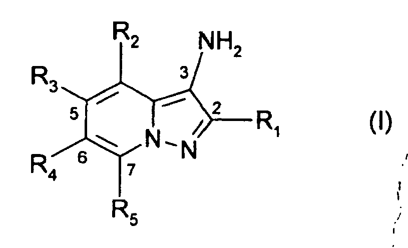
dans laquelle :
- R₁, R₂, R₃, R₄ et R₅, identiques ou différents, représentent un atome d'hydrogène ou d'halogène ; un radical -NHSO₃H ; un radical hydroxyle ; un radical (C₁-C₄)alkyle ; un radical (C₁-C₄)alkoxy ; un radical (C₁-C₄)alkylthio ; mono(C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical nitro ; un radical phényle ; un radical carbonyle ; un radical alkoxy(C₁-C₄)carbonyle ; un radical carboxamido ; un radical cyano ; un radical amino ; un radical sulfonyle; un radical -CO₂H, un radical -SO₃H ; un radical -PO₃H₂ ; un radical -PO₄H₂; ou un groupement de formule (II) suivante : 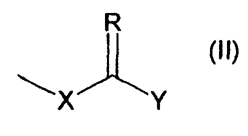 dans lequel R représente un atome d'oxygène ou d'azote, X représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino.

2. Utilisation selon la revendication 1, **caractérisée par le fait que** les composés de formule (I) sont choisis parmi les composés de sous-formule (Ia) suivante, et leurs sels d'addition avec un acide ou avec une base : 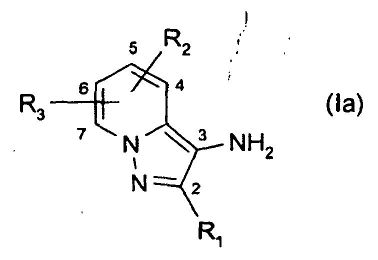
dans laquelle:
R₁, R₂, R₃, identiques ou différents, représentent un atome d'hydrogène ou d'halogène ; un radical hydroxyle ; un radical (C₁-C₄)alkyle ; un radical (C₁-C₄)alkylthio ; un radical (C₁-C₄)alkoxy ; un radical -NHSO₃H ; un radical amino ; un radical (C₁-C₄)alkylamino ; un radical di(C₁-C₄)alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre ; un hétérocycle ; un radical sulfonamide, un radical carbonyle, un radical alcoxy(C₁-C₄)carbonyle, un radical carboxamido, ou un groupement de formule (II) suivante : 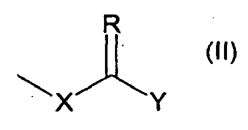 dans lequel R représente un atome d'oxygène ou d'azote, X représente un atome d'oxygène, un groupement NH ou NH(C₁-C₄)alkyle, et Y représente un radical hydroxyle, amino, alkyle en C₁-C₄, (C₁-C₄)alkoxy, (C₁-C₄)alkylamino, ou di(C₁-C₄)alkylamino.

3. Utilisation selon la revendication 1, **caractérisée par le fait que** les 3-amino pyrazolo-[1,5-a]-pyridines de formule (1) sont choisies parmi :
- la pyrazolo[1,5-a]pyridin-3-yiamine ;
- la 2-acétylamino pyrazolo-[1,5-a] pyridin-3-ylamine ;
- la 2-morpholin-4-yl-pyrazolo[1,5-a]pyridin-3-ylamine ;
- l'acide 3-amino-pyrazolo[1,5-a]pyridin-2-carboxylique ;
- la 2-méthoxy-pyrazolo[1,5-a]pyridine-3-ylamino ;
- le (3-amino-pyrazolo[1,5-a]pyridine-7-yl)-méthanol ;
- le 2-(3-amino-pyrazolo[1,5-a]pyridine-5-yl)-éthanol ;
- le 2-(3-amino-pyrazolo[1,5-a]pyridine-7-yl)-éthanol ;
- le (3-amino-pyrazolo[1,5-a]pyridine-2-yl)-méthanol ;
- la 3,6-diamino-pyrazolo[1,5-a]pyridine ;
- la 3,4-diamino-pyrazolo[1,5-a]pyridine ;
- la pyrazolo[1,5-a]pyridine-3,7-diamine ;
- la 7-morpholin-4-yl-pyrazolo[1,5-a]pyridin-3-ylamine ;
- la pyrazolo[1,5-a]pyridine-3,5-diamine ;
- la 5-morpholin-4-yl-pyrazolo[1,5-a]pyridin-3-ylamine ;
- le 2-[(3-amino-pyrazolo[1,5-a]pyridin-5-yl)-(2-hydroxyéthyl)-amino]-éthanol ;
- le 2-[(3-amino-pyrazolo[1,5-a]pyridin-7-yl)-(2-hydroxyéthyl)-amino]-éthanol ;
- la 3-amino-pyrazolo[1,5-a]pyridine-5-ol ;
- la 3-amino-pyrazolo[1,5-a]pyridine-4-ol ;
- la 3-amino-pyrazolo[1,5-a]pyridine-6-ol ;
- la 3-amino-pyrazolo[1,5-a]pyridine-7-ol ;
et leurs d'addition avec un acide ou avec une base.

4. Utilisation selon l'une quelconque des revendications précédentes, **caractérisée par le fait que** la ou les 3-amino pyrazolo-[1,5-a]-pyridines de formule (I) et/ou le ou leurs sels d'addition avec un acide ou une base représentent de 0,0005 à 12 % en poids du poids total de la composition tinctoriale.

5. Utilisation selon la revendication 4, **caractérisée par le fait que** la ou les 3-amino pyrazolo-[1,5-a]-pyridines de formule (I) et/ou le ou leurs sels d'addition avec un acide ou une base représentent de 0,005 à 6 % en poids du poids total de la composition tinctoriale.

6. Utilisation selon l'une quelconque des revendications précédentes, **caractérisée par le fait qu'**elle renferme un ou plusieurs coupleurs choisis parmi les métaphénylènediamines, les méta-aminophénols, les métadiphénols et les coupleurs hétérocycliques.

7. Utilisation selon la revendication 6, **caractérisée par le fait que** les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition.

8. Utilisation selon la revendication 6 ou 7, **caractérisée par le fait que** le ou les coupleurs représentent de 0,0001 à 10 % en poids du poids total de la composition tinctoriale.

9. Utilisation selon la revendication 8, **caractérisée par le fait que** le ou les coupleurs représentent de 0,005 à 5 % en poids du poids total de la composition tinctoriale.

10. Utilisation selon l'une quelconque des revendications précédentes, **caractérisée par le fait qu'**elle contient au moins une base d'oxydation additionnelle choisie parmi les paraphénylènediamines, les bis-phénylalkylènediamines, les para-aminophénols, les ortho-aminophénols et les bases hétérocycliques différentes des 3-amino pyrazolo-[1,5-a]-pyridines de formule (I) telle que définie à la revendication 1.

11. Utilisation selon la revendication 10, **caractérisée par le fait que** la ou les bases d'oxydation additionnelles représentent de 0,0005 à 12 % en poids environ du poids total de la composition tinctoriale.

12. Utilisation selon la revendication 11, **caractérisée par le fait que** la ou les bases d'oxydation additionnelles représentent de 0,005 à 6 % en poids environ du poids total de la composition tinctoriale.

13. Utilisation selon l'une quelconque des revendications précédentes, **caractérisée par le fait que** les sels d'addition avec un acide sont choisis parmi les chlorhydrates, les bromhydrates, les sulfates, les citrates, les succinates, les tartrates, les lactates, les phosphates et les acétates et **par le fait que** les sels d'addition avec une base sont choisis parmi ceux obtenus avec la soude, la potasse, l'ammoniaque ou les amines.

14. Procédé de teinture d'oxydation des fibres kératiniques, **caractérisé par le fait qu'**on applique sur lesdites fibres au moins une composition tinctoriale telle que définie à l'une quelconque des revendications 1 à 13, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

15. Procédé selon la revendication 14, **caractérisé par le fait que** l'agent oxydant présent dans la composition oxydante est choisi parmi le peroxyde d'hydrogène, le peroxyde d'urée, les bromates de métaux alcalins, les persels, les peracides, et les enzymes.

16. 3-amino pyrazolo-[1,5-a]-pyridines de formule (I') suivante, et leurs sels d'addition avec un acide ou avec une base : 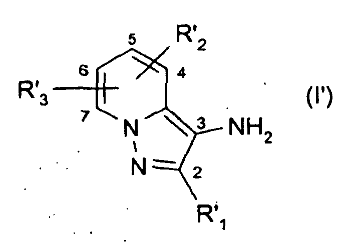
dans laquelle :
- R'₁ représente un atome d'hydrogène ou d'halogène ; un radical hydroxyle ; (C₁-C₄)alkyle ; un radical (C₁-C₄)alkylthio ; un radical (C₁-C₄)alkoxy ; un radical amino ; un radical (C₁-C₄)alkylamino ; un radical di(C₁-C₄)alkylamino dans lequel les deux groupements alkyle, conjointement avec l'atome d'azote auquel ils sont liés, peuvent former un cycle pouvant être interrompu par un ou plusieurs atomes d'azote, d'oxygène ou de soufre ; un hétérocycle ; ou un groupement de formule (II') suivante : 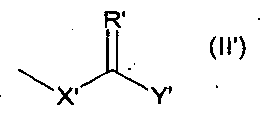 dans lequel R' représente un atome d'oxygène ou d'azote, X' représente un atome d'oxygène, un groupement NH ou NH(C₁-C₄)alkyle, et Y' représente un radical hydroxyle, amino, alkyle en C₁-C₄, (C₁-C₄)alkoxy, (C₁-C₄)alkylamino, ou di(C₁-C₄)alkylamino.
- R'₂ et R'₃, identiques ou différents, représentent un atome d'hydrogène ; un atome d'halogène ; un radical nitro ; un hétérocycle ; un radical NHSO₃H ; un radical suffonamide ; un radical (C₁-C₄)alkyle substitué par un ou plusieurs radicaux identiques ou différents et choisis parmi les hétérocycles, les radicaux -CO₂H, -SO₃H, -PO₃H₂, -PO₄H₂, hydroxyle, tri(C₁-C₄)alkylammonium, -NHSO₃H, sulfonamide, amino, (C₁-C₄)alkylamino et di(C₁-C₄)alkylamino dans lequel les deux radicaux alkyle peuvent former conjointement avec l'atome d'azote auquel ils sont liés un cycle pouvant être interrompu par un ou plusieurs atomes d'azote, de soufre ou d'oxygène ; un radical (C₁-C₄)alkylthio substitué par un ou plusieurs radicaux hydroxyle, amino substitué ou non, ou par un ou plusieurs groupements -CO₂H, -SO₃H, -PO₃H₂ ou PO₄H₂, ou par un ou plusieurs hétérocycles ; un radical (C₁-C₄)alkoxy substitué par un ou plusieurs radicaux hydroxyle, amino substitué ou non, ou par un ou plusieurs groupements -CO₂H, -SO₃H, -PO₃H₂ ou PO₄H₂, ou par un ou plusieurs hétérocycles ; un radical amino substitué par un ou deux radicaux (C₁-C₄)alkyle, ledit ou lesdits radicaux alkyle étant eux-mêmes substitués par un radical amino substitué ou non, tri(C₁-C₄)alkylammonium, -CO₂H, -SO₃H, -PO₃H₂, -PO₄H₂, -NHSO₃H, ou par un hétérocycle ;
étant entendu que :
- au moins un des radicaux R'₁ à R'₃ est différent d'un atome d'hydrogène ;
- les radicaux R'₂ et R'₃ ne peuvent représenter simultanément un atome d'hydrogène ;
- lorsque R'₁ représente un hétérocycle, alors R'₂ et R'₃ sont différents d'un atome d'halogène et d'un atome d'hydrogène ;
- lorsque R'₁ représente un atome d'hydrogène, et que l'un des radicaux R'₂ ou R'₃ représente également un atome d'hydrogène, alors l'autre radical R'₂ ou R'₃ est différent d'un radical hydroxyméthyle en position 7 ou d'un radical β-hydroxyéthyle en position 7 ou 5 ;
- lorsque R'₁ représente un radical méthoxy et que l'un des radicaux R'₂ ou R'₃ représente un atome d'hydrogène, alors l'autre radical R'₂ ou R'₃ est différent d'un atome de chlore.

17. 3-amino pyrazolo-[1,5-a]-pyridines de formule (I') selon la revendication 16, **caractérisées par le fait qu'**elles sont choisies parmi :
- la 5-pyridin-4-yl-pyrazolo[1,5-a]pyridin-3-ylamine ;
- le 4-(3-amino-pyrazolo[1,5-a]pyridin-5-yl)-1-méthyl-pyridinium ;
- le 4-(3-amino-pyrazolo[1,5-a]pyridin-5-yl)-1-(2-hydroxyéthyl)-pyridinium ;
- le (3-amino-pyrazolo[1,5-a]pyridin-2-yl)-pyridin-2-yl-méthanol ;
- le 2-[(3-amino-pyrazolo[1,5-a]pyridin-2-yl)-hydroxyméthyl]-1-méthyl-pyridinium ;
- le 2-[(3-amino-pyrazolo[1,5-a]pyridin-2-yl)-hydroxyméthyl]-1-(2-hydroxyéthyl)-pyridinium ;
- la N7-(2-imidazo-1-yl-propyl)-pyrazolo[1,5-a]pyridin-3,7-diamine ;
- le 3-[2-(3-amino-pyrazolo[1,5-a]pyridin-7-ylamino)-propyl]-1-méthyl-3H-imidazol-1-ium ;
- le 3-[2-(3-amino-pyrazolo[1,5-a]pyridin-7-ylamino)-propyl]-1-(2-hydroxyéthyl)-3H-imidazol-1-ium ;
- la N5-(3-imidazo-1-yl-propyl)-pyrazolo[1,5-a]pyridin-3,5-diamine ;
- le 3-[3-(3-amino-pyrazolo[1,5-a]pyridin-5-ylamino)-propyl]-1-méthyl-3H-imidazol-1-ium ;
- le 3-[3-(3-amino- pyrazolo[1,5-a]pyridin-5-ylamino)-propyl]-1-(2-hydroxyéthyl)-3H-imidazol-1-ium ;
et leurs sels d'addition avec un acide ou avec une base.

18. 3-amino pyrazolo-[1,5-a]-pyridines selon la revendication 16 ou 17, **caractérisées par le fait que** les sels d'addition avec un acide sont choisis parmi les chlorhydrates, les bromhydrates, les sulfates, les citrates, les succinates, les tartrates, les lactates, les phosphates et les acétates et **par le fait que** les sels d'addition avec une base sont choisis parmi ceux obtenus avec la soude, la potasse, l'ammoniaque ou les amines.

19. Composition de teinture des fibres kératiniques comprenant dans un milieu approprié des 3-amino pyrazolo-[1,5]-pyridines de formule (I'), telles que définies à l'une quelconques des revendications 16 à 18, à titre de base d'oxydation.

20. Dispositif à plusieurs compartiments, ou "kit"' de teinture à plusieurs compartiments, dont un premier compartiment renferme une composition tinctoriale telle que définie à la revendication 19 et un second compartiment renferme une composition oxydante.

## Patentansprüche

1. Verwendung einer Zusammensetzung, die in einem zum Färben geeigneten Medium als Oxidationsbase mindestens ein 3-Amino-pyrazolo-[1,5-a]-pyridin der folgenden Formel (I) und/oder eines seiner Additionssalze mit einer Säure oder mit einer Base enthält, zum oxidativen Färben von Keratinfasern: 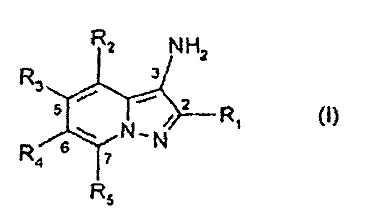
worin bedeuten:
- die Gruppen R₁, R₂, R₃, R₄ und R₅, die gleich oder verschieden sind, ein Wasserstoffatom oder ein Halogenatom; -NHSO₃H; Hydroxy; (C₁₋₄)Alkyl; (C₁₋₄)Alkoxy; (C₁₋₄)Alkylthio; Mono(C₁₋₄)alkylamino; Di(C₁₋₄)alkylamino, wobei die beiden Alkylgruppen gemeinsam mit dem Stickstoffatom, an das sie gebunden sind, einen Ring bilden können, der durch ein oder mehrere Stickstoffatome, Sauerstoffatome oder Schwefelatome unterbrochen sein kann; einen Heterocyclus; Nitro; Phenyl; Carbonyl; Alkoxy(C₁₋₄)carbonyl; Carboxamido; Cyano; Amino; Sulfonyl; -CO₂H; -SO₃H; -PO₃H₂; -PO₄H₂; oder eine Gruppe der folgenden Formel (II): 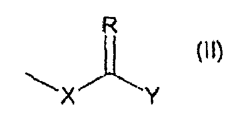 worin die Gruppe R ein Sauerstoffatom oder ein Stickstoffatom bedeutet, X ein Sauerstoffatom, die Gruppe NH oder NH(C₁₋₄)alkyl bedeutet und Y eine Hydroxgruppe, Amino, C₁₋₄-Alkyl, (C₁₋₄)Alkoxy; (C₁₋₄)Alkylamino oder Di(C₁₋₄)alkylamino ist.

2. Verwendung nach Anspruch 1, **dadurch gekennzeichnet, dass** die Verbindungen der Formel (I) unter den Verbindungen der folgenden Formel (Ia) und deren Additionssalzen mit einer Säure oder mit einer Base ausgewählt sind: 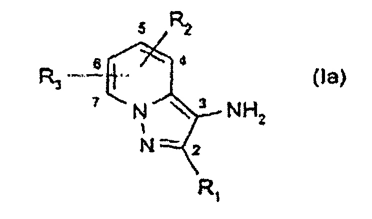
worin bedeuten:
die Gruppen R₁, R₂, R₃, die gleich oder verschieden sind, ein Wasserstoffatom oder ein Halogenatom; Hydroxy; (C₁₋₄)Alkyl; (C₁₋₄)Alkylthio; (C₁₋₄)Alkoxy; -NHSO₃H; Amino; (C₁₋₄)Alkylamino; Di(C₁₋₄)alkylamino, worin die beiden Alkylgruppen gemeinsam mit dem Stickstoffatom, an das sie gebunden sind, einen Ring bilden können, der durch ein oder mehrere Stickstoffatome, Sauerstoffatome oder Schwefelatome unterbrochen sein kann; einen Heterocyclus; Sulfonamid, Carbonyl, Alkoxy(C₁₋₄)carbonyl; Carboxamido oder eine Gruppe der folgenden Formel (II): 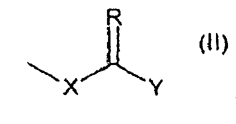 worin die Gruppe R ein Sauerstoffatom oder ein Stickstoffatom bedeutet, X ein Sauerstoffatom, NH oder NH(C₁₋₄)alkyl ist und Y eine Hydroxygruppe, Amino, C₁₋₄-Alkyl, (C₁₋₄)Alkoxy, (C₁₋₄)Alkylamino oder Di(C₁₋₄)alkylamin bedeutet.

3. Verwendung nach Anspruch 1, **dadurch gekennzeichnet, dass** sie 3-Amino-pyrazolo-[1,5-a]-pyridine der Formel (I) ausgewählt sind unter:
- Pyrazolo[1,5-a]pyridin-3-yl-amin;
- 2-Acetylamino-pyrazolo[1,5-a]pyridin-3-yl-amin;
- 2-Morpholin-4-yl-pyrazolo[1,5-a]pyridin-3-yl-amin;
- 3-Amino-pyrazolo[1,5-a]pyridin-2-carbonsäure;
- 2-Methoxy-pyrazolo[1,5-a]pyridin-3-yl-amin;
- (3-Amino-pyrazolo[1,5-a]pyridin-7-yl)-methanol;
- 2-(3-Amino-pyrazolo[1,5-a]pyridin-5-yl)-ethanol;
- 2-(3-Amino-pyrazolo[1,5-a]pyridin-7-yl)-ethanol;
- (3-Amino-pyrazolo[1,5-a]pyridin-2-yl)-methanol;
- 3,6-Diamino-pyrazolo[1,5-a]pyridin;
- 3,4-Diamino-pyrazolo[1,5-a]pyridin;
- Pyrazolo[1,5-a]pyridin-3,7-diamin;
- 7-Morpholin-4-yl-pyrazolo[1,5-a]pyridin-3-yl-amin;
- Pyrazolo[1,5-a]pyridin-3,5-diamin;
- 5-Morpholin-4-yl-pyrazolo[1,5-a]pyridin-3-yl-amin;
- 2-[(3-Arnino-pyrazolo[1,5-a]pyridin-5-yl)-(2-hydroxyethyl)-amino]-ethanol;
- 2-[(3-Amino-pyrazolo[1,5-a]pyridin-7-yl)-(2-hydroxyethyl)-amino]-ethanol;
- 3-Amino-pyrazolo[1,5-a]pyridin-5-ol;
- 3-Amino-pyrazolo[1,5-a]pyridin-4-ol;
- 3-Amino-pyrazolo[1,5-a]pyridin-6-ol;
- 3-Amino-pyrazolo[1,5-a]pyridin-7-ol;
und deren Additionssalze mit einer Säure oder einer Base.

4. Verwendung nach einem der vorhergehenden Ansprüche, **dadurch gekennzeichnet, dass** das oder die 3-Amino-pyrazolo-[1,5-a]-pyridin(e) der Formel (I) und/oder deren Additionssalz(e) mit einer Säure oder einer Base 0,005 bis 12 Gew.-% des Gesamtgewichts der Farbmittelzusammensetzung ausmachen.

5. Verwendung nach Anspruch 4, **dadurch gekennzeichnet, dass** das oder die 3-Amino-pyrazolo-[1,5-a]pyridin(e) der Formel (I) und/oder deren Additionssalz(e) mit einer Säure oder einer Base 0,005 bis 6 Gew.-% des Gesamtgewichts der Farbmittelzusammensetzung ausmachen.

6. Verwendung nach einem der vorhergehenden Ansprüche, **dadurch gekennzeichnet, dass** sie einen oder mehrere Kuppler enthält, die unter den m-Phenylendiaminen, m-Aminophenolen, m-Dihydroxybenzolen und den hetereocyclischen Kupplern ausgewählt ist.

7. Verwendung nach Anspruch 6, **dadurch gekennzeichnet, dass** die Kuppler unter 2-Methyl-5-aminophenol, 5-N-(β-Hydroxyethyl)amino-2-methylphenol, 3-Aminophenol, 1,3-Dihydroxybenzol, 1,3-Dihydroxy-2-methylbenzol, 4-Chlor-1,3-dihydroxybenzol, 2,4-Diamino-1-(β-hydroxyethyloxy)benzol, 2-Amino-4-(β-hydroxyethylamino-1-methoxybenzol, 1,3-Diaminobenzol, 1,3-Bis-(2,4-diaminophenoxy)propan, Sesamol, α-Naphthol, 2-Methyl-1-naphthol, 6-Hydroxyindol, 4-Hydroxyindol, 4-Hydroxy-N-methylindol, 6-Hydroxyindolin, 2,6-Dihydroxy-4-methylpyridin, 1-H-3-Methylpyrazol-5-on, 1-Phenyl-3-methylpyrazol-5-on und deren Additionssalzen ausgewählt sind.

8. Verwendung nach Anspruch 6 oder 7, **dadurch gekennzeichnet, dass** der oder die Kuppler 0,0001 bis 10 Gew.-% des Gesamtgewichts der Farbmittelzusammensetzung ausmachen.

9. Verwendung nach Anspruch 8, **dadurch gekennzeichnet, dass** der oder die Kuppler 0,005 bis 5 Gew.-% des Gesamtgewichts der Farbmittelzusammensetzung ausmachen.

10. Verwendung nach einem der vorhergehenden Ansprüche, **dadurch gekennzeichnet, dass** sie ferner mindestens eine zusätzliche Oxidationsbase enthält, die unter den p-Phenylendiaminen, Bisphenylalkylendiaminen, p-Aminophenolen, o-Aminophenolen und den heterocyclischen Basen ausgewählt ist, die von den 3-Amino-pyrazolo-[1,5-a]-pyridinen der Formel (I) nach Anspruch 1 verschieden sind.

11. Verwendung nach Anspruch 10, **dadurch gekennzeichnet, dass** die zusätzliche(n) Oxidationsbase(n) etwa 0,0005 bis 12 Gew.-% des Gesamtgewichts der Farbmittelzusammensetzung ausmachen.

12. Verwendung nach Anspruch 11, **dadurch gekennzeichnet, dass** die zusätzliche(n) Oxidationsbase(n) etwa 0,005 bis 6 Gew.-% des Gesamtgewichts der Farbmittelzusammensetzung ausmachen.

13. Verwendung nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Lactaten, Phosphaten und Acetaten ausgewählt sind und die Additionssalze mit einer Base unter den Salzen ausgewählt sind, die mit Natriumhydroxid, Kaliumhydroxid, Ammoniak oder Aminen gebildet werden.

14. Verfahren zum oxidativen Färben von Keratinfasern, **dadurch gekennzeichnet, dass** auf die Fasern mindestens eine Farbmittelzusammensetzung nach einem der Ansprüche 1 bis 13 aufgetragen und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

15. Verfahren nach Anspruch 14, **dadurch gekennzeichnet, dass** das Oxidationsmittel in einer oxidierenden Zusammensetzung vorliegt, die unter Wasserstoffperoxid, Harnstoffperoxid, Alkalimetallbromaten, Persalzen, Persäuren und Enzymen ausgewählt ist.

16. 3-Amino-pyrazolo-[1,5-a] -pyridine der folgenden Formel (I') und deren Additionssalze mit einer Säure oder mit einer Base: 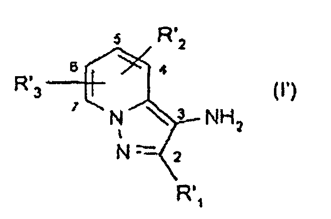
worin bedeuten:
- die Gruppe R'₁ ein Wasserstoffatom oder ein Halogenatom; Hydroxy; (C₁₋₄)Alkyl; (C₁₋₄)Alkylthio; (C₁₋₄)Alkoxy; Amino; (C₁₋₄)Alkylamino; Di(C₁₋₄)alkylamino, worin die beiden Alkylgruppen gemeinsam mit dem Stickstoffatom, an das sie gebunden sind, einen Ring bilden können, der durch ein oder mehrere Stickstoffatome, Sauerstoffatome oder Schwefelatome unterbrochen sein kann; einen Heterocyclus; oder eine Gruppe der folgenden Formel (II'): 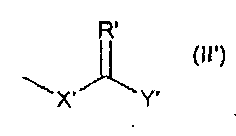 worin R' ein Sauerstoffatom oder Stickstoffatom bedeutet, X' ein Sauerstoffatom, eine Gruppe NH oder NH(C₁₋₄)alkyl ist und Y' Hydroxy, Amino, C₁₋₄-Alkyl, (C₁₋₄)Alkoxy, (C₁₋₄)Alkylamino oder Di(C₁₋₄)alkylamino bedeutet;
- die Gruppen R'₂ und R'₃, die gleich oder verschieden sind, ein Wasserstoffatom; ein Halogenatom; Nitro; einen Heterocyclus; NHSO₃H; Sulfonamid; eine (C₁₋₄)Alkylgruppe, die mit einer oder mehreren Gruppen substituiert ist, die gleich oder verschieden sind, und unter den Heterocyclen, -CO₂H, -SO₃H, -PO₃H₂, -PO₄H₂, Hydroxy, Tri(C₁₋₄)alkylammonium, -NHSO₃H, Sulfonamid, Amino, (C₁₋₄)alkylamino und Di(C₁₋₄)alkylamino ausgewählt sind, worin die beiden Alkylgruppen gemeinsam mit dem Stickstoffatom, an das sie gebunden sind, einen Ring bilden können, der durch ein oder mehrere Stickstoffatome, Schwefelatome oder Sauerstoffatome unterbrochen sein kann; eine (C₁₋₄)Alkylaminogruppe, die mit einer oder mehreren Hydroxygruppen, unsubstituierten oder substituierten Aminogruppen oder einer oder mehreren Gruppen -CO₂H, -SO₃H, -PO₃H₂ oder PO₄H₂ oder einem oder mehreren Heterocyclen substituiert ist; eine (C₁₋₄)Alkoxygruppe, die mit einer oder mehreren der folgenden Gruppen substituiert ist: Hydroxy, unsubstituierten oder substituierten Aminogruppen oder einer oder mehreren Gruppen -CO₂H, -SO₃H, -PO₃H₂ oder -PO₄H₂ oder einem oder mehreren Heterocyclen; eine Aminogruppe, die mit einer oder zwei (C₁₋₄)Alkylgruppen substituiert ist, wobei die Alkylgruppe(n) selbst mit einer substituierten oder unsubstituierten Aminogruppe, Tri(C₁₋₄)alkylammonium, -CO₂H, -SO₃H, -PO₃H₂ oder -PO₄H₂, NHSO₃H oder einem Heterocyclus substituiert sein kann;
mit der Maßgabe, dass:
- mindestens eine der Gruppen R'₁ bis R'₃ von Wasserstoff verschieden ist;
- die Gruppen R'₂ und R'₃ nicht gleichzeitig Wasserstoff bedeuten können;
- R'₂ und R'₃ von einem Halogenatom und einem Wasserstoffatom verschieden sind, wenn R'₁ einen Heterocyclus bedeutet;
- wenn R'₁ ein Wasserstoffatom bedeutet und eine der Gruppen R'₂ oder R'₃ ebenfalls ein Wasserstoffatom bedeutet, die andere Gruppe R'₂ oder R'₃ von einer Hydroxymethylgruppe in 7-Stellung oder einer β-Hydroxyethyl in 5- oder 7-Stellung verschieden ist;
- wenn R'₁ eine Methoxygruppe bedeutet und eine der Gruppen R'₂ oder R'₃ ein Wasserstoffatom ist, die andere Gruppe R'₂ oder R'₃ von einem Chloratom verschieden ist.

17. 3-Amino-pyrazolo-[1,5-a]-pyridine der Formel (I') nach Anspruch 16, **dadurch gekennzeichnet, dass** sie ausgewählt sind unter:
- 5-Pyridin-4-yl-pyrazolo[1,5-a]pyridin-3-yl-amin;
- 4-(3-Amino-pyrazolo[1,5-a]pyridin-5-yl)-1-methylpyridinium;
- 4-(3-Amino-pyrazolo[1,5-a]pyridin-5-yl)-1-(2-hydroxyethyl)-pyridinium;
- (3-Amino-pyrazolo[1,5-a]pyridin-2-yl)-pyridin-2-yl-methanol;
- 2-[(3-Amino-pyrazolo[1,5-a]pyridin-2-yl)-hydroxymethyl]-1-methylpyridinium;
- 2-[(3-Amino-pyrazolo[1,5-a]pyridin-2-yl)-hydroxymethyl]-1-(2-hydroxyethyl)pyridinium;
- N7-(2-Imidazo-1-yl-propyl)-pyrazolo[1,5-a]pyridin-3,7-diamin;
- 3-[2-(3-Amino-pyrazolo[1,5-a]pyridin-7-ylamino)-propyl]-1-methyl-3H-imidazol-1-ium;
- 3-[2-(3-Amino-pyrazolo[1,5-a]pyridin-7-ylamino)-propyl]-1-(2-hydroxyethyl)-3H-imidazol-1-ium;
- N5-(3-Imidazo-1-yl-propyl)-pyrazolo[1,5-a]pyridin-3,5-diamin;
- 3-[3-(3-Amino-pyrazolo[1,5-a]pyridin-5-ylamino)-propyl]-1-methyl-3H-imidazol-1-ium;
- 3-[3-(3-Amino-pyrazolo[1,5-a]pyridin-5-ylamino)-propyl]-1-(2-hydroxyethyl)-3H-imidazol-1-ium;
und deren Additionssalzen mit einer Säure oder mit einer Base.

18. 3-Amino-pyrazolo-[1,5-a]-pyridine nach Anspruch 16 oder 17, **dadurch gekennzeichnet, dass** die Additionssalze mit einer Säure unter den Hydrochloriden, Hydrobromiden, Sulfaten, Citraten, Succinaten, Tartraten, Lactaten, Phosphaten und Acetaten und die Additionssalze mit einer Base unter den Salzen ausgewählt sind, die mit Natriumhydroxid, Kaliumhydroxid, Ammoniak oder Aminen gebildet werden.

19. Zusammensetzung zum Färben von Keratinfasern, die in einem zum Färben geeigneten Medium 3-Amino-pyrazolo-[1,5]-pyridine der Formel (I') nach einem der Ansprüche 16 bis 18 als Oxidationsbase enthält.

20. Vorrichtung mit mehreren Abteilungen oder Kit zum Färben mit mehreren Abteilungen, wobei eine erste Abteilung eine Farbmittelzusammensetzung nach Anspruch 19 und eine zweite Abteilung eine oxidierende Zusammensetzung enthält.

## Claims

1. Use for the oxidation dyeing of keratinous fibres of a composition comprising, in a medium appropriate for dyeing, at least one 3-aminopyrazolo[1,5-a]pyridine of formula (I) below as oxidation base and/or one of its addition salts with an acid or with a base: 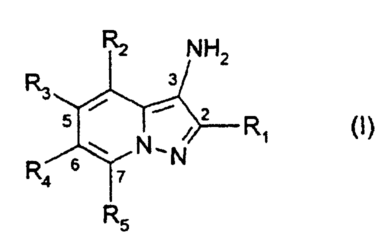
in which:
- R₁, R₂, R₃, R₄ and R₅, which are identical or different, represent a hydrogen or halogen atom; an -NHSO₃H radical; a hydroxyl radical; a (C₁-C₄)alkyl radical; a (C₁-C₄)alkoxy radical; a (C₁-C₄)alkylthio radical; mono (C₁-C₄) alkylamino; a di (C₁-C₄) alkylamino radical in which the two alkyl groups, in conjunction with the nitrogen atom to which they are bonded, may form a ring which may be interrupted by one or more nitrogen, oxygen or sulphur atoms; a heterocycle; a nitro radical; a phenyl radical; a carbonyl radical; a ((C₁-C₄)alkoxy)carbonyl radical; a carboxamido radical; a cyano radical; an amino radical; a sulphonyl radical; a -CO₂H radical, an -SO₃H radical; a -PO₃H₂ radical; a -PO₄H₂ radical; or a group of formula (II) below: 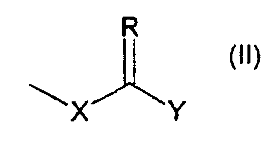 in which R represents an oxygen or nitrogen atom, X represents an oxygen atom or an NH or NH(C₁-C₄)alkyl group, and Y represents a hydroxyl, amino, C₁-C₄ alkyl, (C₁-C₄)alkoxy, (C₁-C₄)alkylamino or di(C₁-C₄)alkylamino radical.

2. Use according to Claim 1, **characterized in that** the compounds of formula (I) are selected from compounds of subformula (Ia) below and their addition salts with an acid or with a base: 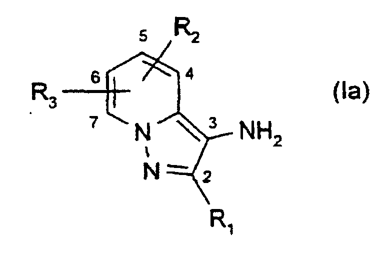
in which:
R₁, R₂ and R₃, which are identical or different, represent a hydrogen or halogen atom; a hydroxyl radical; a (C₁-C₄)alkyl radical; a (C₁-C₄) alkylthio radical; a (C₁-C₄)alkoxy radical; an -NHSO₃H radical; an amino radical; a (C₁-C₄)alkylamino radical; a di(C₁₋C₄)alkylamino radical in which the two alkyl groups, in conjunction with the nitrogen atom to which they are bonded, may form a ring which may be interrupted by one or more nitrogen, oxygen or sulphur atoms; a heterocycle; a sulphonamide radical; a carbonyl radical; a ((C₁-C₄)alkoxy)carbonyl radical; a carboxamido radical; or a group of formula (II) below: 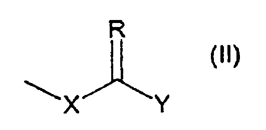 in which R represents an oxygen or nitrogen atom, X represents an oxygen atom or an NH or NH(C₁-C₄)alkyl group, and Y represents a hydroxyl, amino, C₁-C₄ alkyl, (C₁-C₄)alkoxy, (C₁-C₄)alkylamino or di(C₁-C₄)alkylamino radical.

3. Use according to Claim 1, **characterized in that** the 3-aminopyrazolo[1,5-a]pyridines of formula (I) are selected from:
- pyrazolo[1,5-a]pyridin-3-ylamine;
- 2-acetylaminopyrazolo[1,5-a]pyridin-3-ylamine;
- 2-morpholin-4-ylpyrazolo[1,5-a]pyridin-3-ylamine;
- 3-aminopyrazolo[1,5-a]pyridine-2-carboxylic acid;
- 2-methoxypyrazolo[1,5-a]pyridin-3-ylamine;
- (3-aminopyrazolo[1,5-a]pyridin-7-yl)methanol;
- 2-(3-aminopyrazolo[1,5-a]pyridin-5-yl)ethanol;
- 2-(3-aminopyrazolo[1,5-a]pyridin-7-yl)ethanol;
- (3-aminopyrazolo[1,5-a]pyridin-2-yl)methanol;
- 3,6-diaminopyrazolo[1,5-a]pyridine;
- 3,4-diaminopyrazolo[1,5-a]pyridine;
- pyrazolo[1,5-a]pyridine-3,7-diamine;
- 7-morpholin-4-ylpyrazolo[1,5-a]pyridin-3-ylamine;
- pyrazolo[1,5-a]pyridine-3,5-diamine;
- 5-morpholin-4-ylpyrazolo[1,5-a]pyridin-3-ylamine;
- 2-[(3-aminopyrazolo[1,5-a]pyridin-5-yl)-(2-hydroxyethyl)amino]ethanol;
- 2-[(3-aminopyrazolo[1,5-a]pyridin-7-yl)-(2-hydroxyethyl)amino]ethanol;
- 3-aminopyrazolo[1,5-a]pyridin-5-ol;
- 3-aminopyrazolo[1,5-a]pyridin-4-ol;
- 3-aminopyrazolo[1,5-a]pyridin-6-ol;
- 3-aminopyrazolo[1,5-a]pyridin-7-ol;
and their addition salts with an acid or with a base.

4. Use according to any one of the preceding claims, **characterized in that** the 3-aminopyrazolo[1,5-a]pyridine(s) of formula (I) and/or its or their addition salts with an acid or a base make(s) up from 0.0005 to 12% by weight of the total weight of the dyeing composition.

5. Use according to Claim 4, **characterized in that** the 3-aminopyrazolo[1,5-a]pyridine(s) of formula (I) and/or its or their addition salts with an acid or a base make(s) up from 0.005 to 6% by weight of the total weight of the dyeing composition.

6. Use according to any one of the preceding claims, **characterized in that** the composition comprises one or more couplers selected from meta-phenylenediamines, meta-aminophenols, meta-diphenols and heterocyclic couplers.

7. Use according to Claim 6, **characterized in that** the couplers are selected from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 2-methyl-1-naphthol, 6-hydroxyindole, 4-hydroxyindole, 4-hydroxy-N-methylindole, 6-hydroxyindoline, 2,6-dihydroxy-4-methylpyridine, 1H-3-methylpyrazol-5-one, 1-phenyl-3-methylpyrazol-5-one, and their addition salts.

8. Use according to Claim 6 or 7, **characterized in that** the coupler(s) make(s) up from 0.0001 to 10% by weight of the total weight of the dyeing composition.

9. Use according to Claim 8, **characterized in that** the coupler(s) make(s) up from 0.005 to 5% by weight of the total weight of the dyeing composition.

10. Use according to any one of the preceding claims, **characterized in that** the composition comprises at least one additional oxidation base selected from para-phenylenediamines, bisphenylalkylenediamines, para-aminophenols, ortho-aminophenols and heterocyclic bases other than the 3-aminopyrazolo[1,5-a]pyridines of formula (I) as defined in Claim 1.

11. Use according to Claim 10, **characterized in that** the additional oxidation base(s) make(s) up from 0.0005 to 12% by weight approximately of the total weight of the dyeing composition.

12. Use according to Claim 11, **characterized in that** the additonal oxidation base(s) make(s) up from 0.005 to 6% by weight approximately of the total weight of the dyeing composition.

13. Use according to any one of the preceding claims, **characterized in that** the addition salts with an acid are selected from hydrochlorides, hydrobromides, sulphates, citrates, succinates, tartrates, lactates, phosphates and acetates and **in that** the addition salts with a base are selected from those obtained with sodium hydroxide, potassium hydroxide, aqueous ammonia or amines.

14. Method of oxidation dyeing of keratinous fibres, **characterized in that** at least one dyeing composition as defined in any one of Claims 1 to 13 is applied to said fibres and **in that** the colour is developed at acid, neutral or alkaline pH by means of an oxidizing agent which is added to the dyeing composition at the time of use or which is present in an oxidizing composition applied simultaneously or sequentially.

15. Method according to Claim 14, **characterized in that** the oxidizing agent present in the oxidizing composition is selected from hydrogen peroxide, urea peroxide, alkali metal bromates, persalts, peracids and enzymes.

16. 3-Aminopyrazolo[1,5-a]pyridines of formula (I') below and their addition salts with an acid or with a base: 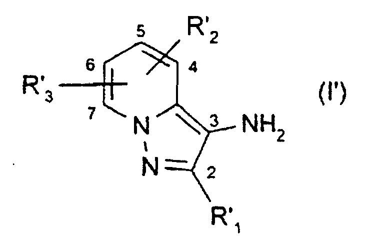
in which:
- R'₁ represents a hydrogen or halogen atom; a hydroxyl radical; (C₁-C₄)alkyl; a (C₁-C₄) alkylthio radical; a (C₁-C₄)alkoxy radical; an amino radical; a (C₁-C₄)alkylamino radical; a di(C₁-C₄)alkylamino radical in which the two alkyl groups, in conjunction with the nitrogen atom to which they are bonded, may form a ring which may be interrupted by one or more nitrogen, oxygen or sulphur atoms; a heterocycle; or a group of formula (II') below: 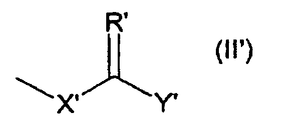 in which R' represents an oxygen or nitrogen atom, X' represents an oxygen atom or an NH or NH(C₁-C₄)alkyl group, and Y' represents a hydroxyl, amino, C₁-C₄ alkyl, (C₁-C₄)alkoxy, (C₁-C₄)alkylamino or di(C₁-C₄)alkylamino radical;
- R'₂ and R'₃, which are identical or different, represent a hydrogen atom; a halogen atom; a nitro radical; a heterocycle; an NHSO₃H radical; a sulphonamide radical; a (C₁-C₄)alkyl radical substituted by one or more identical or different radicals selected from heterocycles, -CO₂H, -SO₃H, -PO₃H₂, -PO₄H₂, hydroxyl, tri (C₁-C₄) alkylammonium, -NHSO₃H, sulphonamide, amino, (C₁-C₄)alkylamino and di(C₁₋C₄)alkylamino radicals in which the two alkyl radicals, in conjunction with the nitrogen atom to which they are bonded, may form a ring which may be interrupted by one or more nitrogen, sulphur or oxygen atoms; a (C₁₋C₄)alkylthio radical substituted by one or more hydroxyl or substituted or unsubstituted amino radicals or by one or more -CO₂H, -SO₃H, -PO₃H₂ or -PO₄H₂ groups or by one or more heterocycles; a (C₁-C₄)alkoxy radical substituted by one or more hydroxyl or substituted or unsubstituted amino radicals or by one or more -CO₂H, -SO₃H, -PO₃H₂ or -PO₄H₂ groups or by one or more heterocycles; an amino radical substituted by one or two (C₁-C₄)alkyl radicals, said alkyl radical or radicals being itself or themselves substituted by a substituted or unsubstituted amino, tri(C₁₋C₄)alkylammonium, -CO₂H, -SO₃H, -PO₃H₂, -PO₄H₂ or -NHSO₃H radical or by a heterocycle;
with the proviso that:
- at least one of the radicals R'₁ to R'₃ is other than a hydrogen atom;
- the radicals R'₂ and R'₃ cannot simultaneously represent a hydrogen atom;
- when R'₁ represents a heterocycle, R'₂ and R'₃ are other than a halogen atom and a hydrogen atom;
- when R'₁ represents a hydrogen atom and when one of the radicals R'₂ or R'₃ also represents a hydrogen atom, the other radical R'₂ or R'₃ is other than a hydroxymethyl radical in position 7 or than a β-hydroxyethyl radical in position 7 or 5;
- when R'₁ represents a methoxy radical and when one of the radicals R'₂ or R'₃ represents a hydrogen atom, the other radical R'₂ or R'₃ is other than a chlorine atom.

17. 3-Aminopyrazolo[1,5-a]pyridines of formula (I') according to Claim 16, **characterized in that** they are selected from:
- 5-pyridin-4-ylpyrazolo[1,5-a]pyridin-3-ylamine;
- 4-(3-aminopyrazolo[1,5-a]pyridin-5-yl)-1-methylpyridinium;
- 4-(3-aminopyrazolo[1,5-a]pyridin-5-yl)-1-(2-hydroxyethyl)pyridinium;
- (3-aminopyrazolo[1,5-a]pyridin-2-yl)pyridin-2-ylmethanol;
- 2-[(3-aminopyrazolo[1,5-a]pyridin-2-yl)hydroxymethyl]-1-methylpyridinium;
- 2-[(3-aminopyrazolo[1,5-a]pyridin-2-yl)hydroxymethyl]-1-(2-hydroxyethyl)pyridinium;
- N-7-(2-imidazol-1-ylpropyl)pyrazolo[1,5-a]pyridine-3,7-diamine;
- 3-[2-(3-aminopyrazolo[1,5-a]pyridin-7-ylamino)propyl]-1-methyl-3H-imidazol-1-ium;
- 3-[2-(3-aminopyrazolo[1,5-a]pyridin-7-ylamino)propyl]-1-(2-hydroxyethyl)-3H-imidazol-1-ium;
- N-5-(3-imidazol-1-ylpropyl)pyrazolo[1,5-a]pyridine-3,5-diamine;
- 3-[3-(3-aminopyrazolo[1,5-a]pyridin-5-ylamino)propyl]-1-methyl-3H-imidazol-1-ium;
- 3-[3-(3-aminopyrazolo[1,5-a]pyridin-5-ylamino)propyl]-1-(2-hydroxyethyl)-3H-imidazol-1-ium;
and their addition salts with an acid or with a base.

18. 3-Aminopyrazolo[1,5-a]pyridines according to Claim 16 or 17, **characterized in that** the addition salts with an acid are selected from hydrochlorides, hydrobromides, sulphates, citrates, succinates, tartrates, lactates, phosphates and acetates and **in that** the addition salts with a base are selected from those obtained with sodium hydroxide, potassium hydroxide, aqueous ammonia or amines.

19. Composition for dyeing keratinous fibres comprising, in an appropriate medium, 3-aminopyrazolo[1,5-a]pyridines of formula (I') as defined in any one of Claims 16 to 18 as oxidation bases.

20. Multicompartment dyeing device or kit of which a first compartment contains a dyeing composition as defined in Claim 19 and a second compartment contains an oxidizing composition.
